# EUROPEAN PATENT APPLICATION

(11) **EP 4 008 378 A1**
(43) Date of publication of application: **08.06.2022**
(21) Application number: 20757944.2
(22) Date of filing: 01.07.2020
(51) Int. Cl.: A61M 5/19, A61M 5/28, A61M 5/31, A61M 5/315

(54) **DUAL-CHAMBER SYRINGE**

(30) Priority: 05.07.2019 PT 2019115633
(71) Applicant: Escola Superior de Enfermagem de Coimbra, 3001-901 Coimbra (PT); Muroplas - Industria de Plasticos, S.A., 4745-334 Muro - Trofa (PT); Piep - Pólo De Inovação em Engenharia de Polímeros, 4800-058 Guimarães (PT)
(72) Inventor: SANTOS DINIS PARREIRA, Pedro Miguel, 3045-293 Coimbra (PT); DE SOUSA SALGUEIRO OLIVEIRA, Anabela, 3030-175 Coimbra (PT); GUARDADO CRUZ, Arménio, 3030-393 Coimbra (PT); BAPTISTA SOUSA, Liliana, 2435-681 Urqueira (PT); FIGUEIRA MARQUES, Inês Alexandra, 3500-541 Silgueiros, Viseu (PT); GOMES ROQUE, Ana Filipa, 3045-160 Coimbra (PT); FERNANDES, Tânia, 8005-512 Faro (PT); MELO COELHO FERREIRA, Jorge Manuel, 4785-333 Trofa (PT); COUTO CORTEZ, Sara Isabel, 4770-369 Vila Nova De Famalicão (PT); SANTOS COELHO, Alberta Augusta, 4745-889 São Mamede De Negrelos (PT); DA SILVA MAIA, André Filipe, 4745-265 Guidões (PT); GONÇALVES DA COSTA CARNEIRO, Ana Filipa, 4810-426 Guimarães (PT); PEREIRA DA SILVA, Bruno Alexandre, 4765-036 Bairro (PT); DUARTE FREITAS, Ricardo Manuel, 4705-479 Braga (PT); ROCHA MARQUES, Ângelo Daniel, 4805-489 Guimarães (PT); FERREIRA BASTOS, Lourenço Manuel, 4700-446 Braga (PT); ARAÚJO MARQUES, Ana Rita, 4700-060 Braga (PT); FARIA DO VALE, Bruno Miguel, 4810-534 Guimarães (PT); DOS SANTOS COSTA, Paulo Jorge, 3080-503 Tavarede (PT)
(74) Representative: Pereira da Cruz, Joao
(86) International application number: PCT/IB2020/056219
(87) International publication number: WO 2021/005459

(57) **Abstract**

This invention relates to a double chamber syringe (1), that is, a chamber comprising two independent compartments with independent outlets, and two independent plungers (2)(3) preventing the mixing of fluids. This invention arises from the need for a syringe for intravenous administration that simultaneously allows the loading and sequential administration of the medicine and the solution for washing the medical device through which the medicine was administered. All the constituents are configured in such a way that they allow the sequential administration of the washing solution (pre-flushing), of the medicine, and again of the washing solution (final flushing).

## Description

### SCOPE OF THE INVENTION

The administration of solutions, whether they are medicines or fluids, intravenously, is a daily activity in clinical practice. In this context, the administration of intravenous medication with a syringe, through peripheral venous catheter (PVC) or central venous catheter (CVC), facilitates the administration of medicines or other fluids directly into the bloodstream.

The use and permanence of the PVC or CVC in the provision of nursing care is not free from complications, both local and systemic ones. These may manifest through signs and/or symptoms of vascular trauma, such as phlebitis, infiltration or extravasation, infection, or malfunction of the catheter due to its obstruction.

In order to prevent the occurrence of the aforementioned complications, and according to the international guidelines, all venous accesses should be evaluated before use as regards their permeability, and a pre-flushing should be performed. Similarly, these venous accesses should be washed between and after administration of intravenous therapy.

Thus, the catheter washing procedure (flushing) allows the catheter permeability to be confirmed before the administration of medicines/fluids (pre-flushing), prevents and/or reduces the associated complications, keeping the catheter's good functioning and, thus, contributes to a reduction of the patient's morbidity and a lower need to replace it due to complications.

Flushing is a recommended nursing intervention, which helps with removing debris from the internal walls of the catheter inserted in the vein, also allowing reducing the deposition of medicines (and consequently any incompatibility reactions in subsequent administrations). It prevents biofilm formation and colonization by microorganisms inside the catheter, in as much as colonization and formation of biofilm can occur within three days of catheterization.

In what concerns the prevention of interaction between incompatible substances and the residual deposition of medicines in the catheter lumen, by ensuring washing after each administration, the risk of complications associated to drug interaction between incompatible solutions is reduced.

However, for a number of reasons, nursing care often culminates in the omission of the flushing process, thus representing a risk factor for the patient's safety. Additionally, in order to comply with this flushing process (before and after administration of the medication), two syringes are required (one to check vascular permeability, and one to perform the flushing after administration of the medication).

The present invention responds to the above-mentioned needs and relates to a medical device, namely a double chamber syringe, which allows the loading and sequential administration of two different fluids (medicine and washing solution), without the need to change syringes, that is, allowing that a single device is used to check the catheter permeability (pre-flushing), administering the medicine and, subsequently, washing the catheter (final flushing).

### DESCRIPTION OF THE FIGURES

The reference numbers relate to the following:
(1) Double chamber with independent outlets/inlets;
(2) Medication chamber independent plunger;
(3) Washing solution chamber independent plunger;
(4) Single outlet selector;
(5) Chamber with dual purpose, functioning as a plunger.

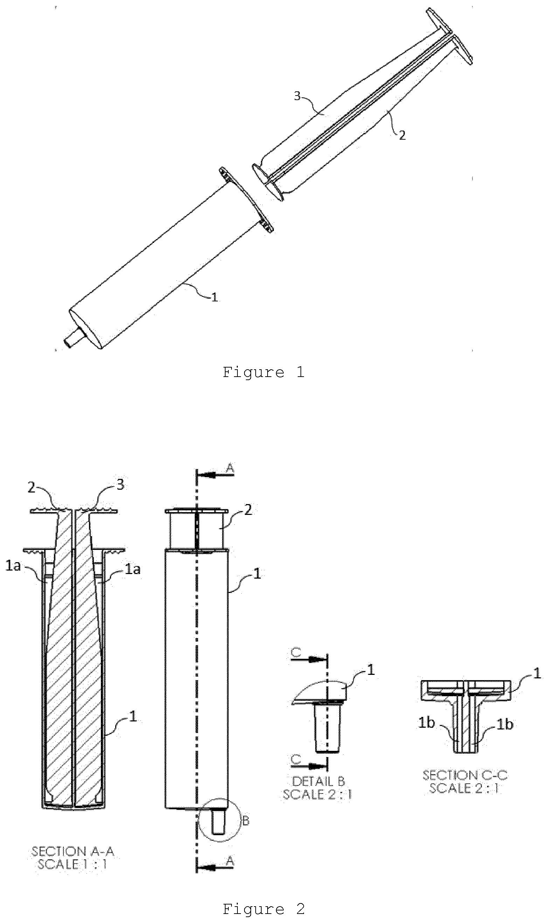

Figures 1, 2, and 3 - Representation of the present invention in its first essential preferential embodiment, consisting of three independent components: the syringe body which is a double chamber (1) and two independent plungers (2)(3). It is provided with two independent outlets (1b), preventing the mixing of fluids. The syringe comprises a double chamber (1) with two compartments (1a) with independent outlets (1b). One chamber is intended for the washing solution and the other chamber for the medicine to be administered, avoiding the mixing of fluids and allowing a sequential administration of the washing solution (pre-flushing), the medicine, and again the washing solution (final flushing).

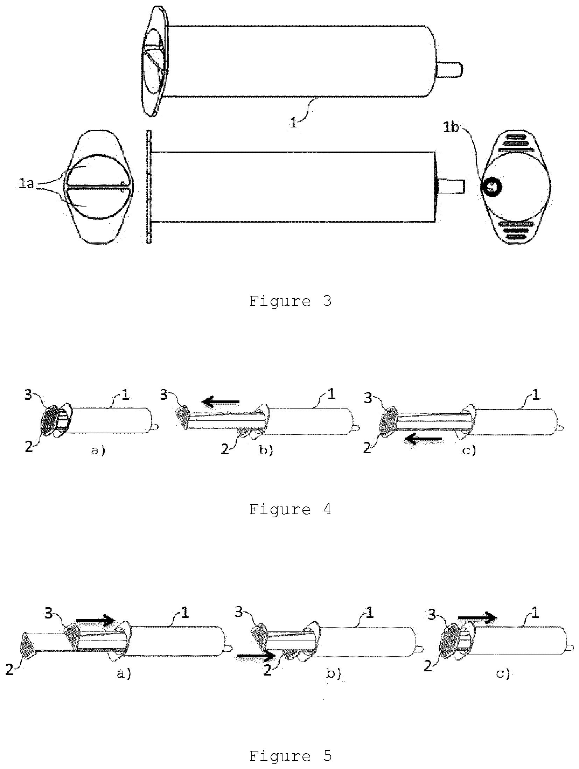

Figure 4 - Representation of the loading phase of the preferential embodiment shown in Figures 1, 2 and 3. The syringe is placed in the initial position, as in Fig. 4- a), and the loading phase is carried out using two movements, loading the washing solution chamber by moving the plunger (3) in the direction indicated in Fig. 4b) and loading the medicine chamber by moving the plunger (2) in the direction indicated in Fig. 4c).
Figure 5 - Representation of the administration phase in the preferential embodiment shown in Figures 1, 2 and 3, this phase being achieved in three different movements. The pre-flushing with washing solution is performed by moving the washing solution plunger (3) until completing the assessment of the catheter's permeability (pre-flushing) (Fig. 5a). The second movement is performed for administration of the medicine by moving the plunger (2) until the end of the total capacity of the respective chamber (1a), Fig. 5b). Finally, the washing solution plunger (3) is moved again for the final flushing, Fig. 5c).

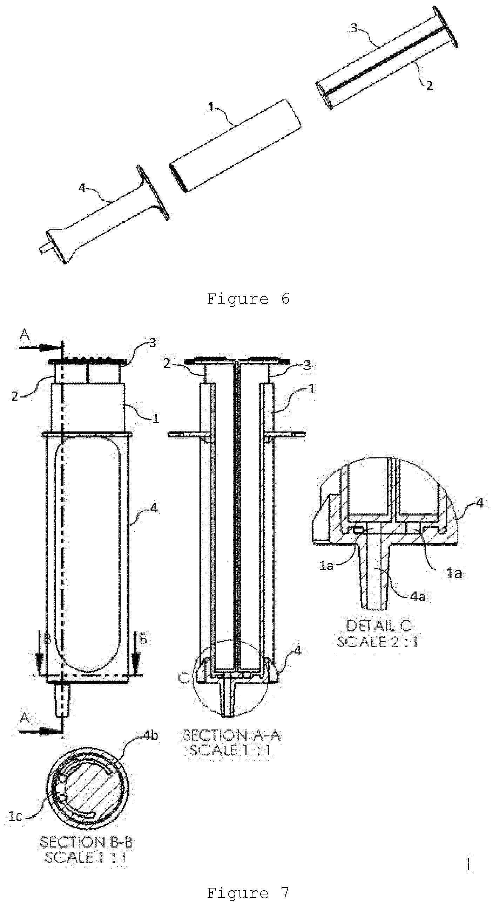

Figures 6 and 7 - Representation of this invention in a second preferential embodiment consisting of four independent components, a selector (4), a double chamber (1) and two independent plungers (2 and 3). The chamber (1) has two compartments (1a) and two independent outlets (1b) preventing the mixing of fluids, and the selector (4), with only one outlet, makes it possible to alternate between the washing solution and the medicine. The concept presented allows an administration that includes pre-flushing, medication administration and final flushing. The syringe, in this preferential embodiment, comprises a double chamber (1) with independent outlets (1b). One chamber is intended for the washing solution and the other chamber for the medicine to be administered, avoiding the mixing of fluids and allowing a sequential administration of the washing solution (pre-flushing), then the medicine, and again the washing solution (final flushing). The chamber has an embossing (1c) that corresponds to the path to be taken when the selector (4) is rotated; the latter, in its turn, has a recess (4b) that allows the position to be selected depending on whether the washing solution or the medicine is loaded/administered. In the selector (4), the washing solution and the medicine are loaded and/or administered through the same outlet (4a).

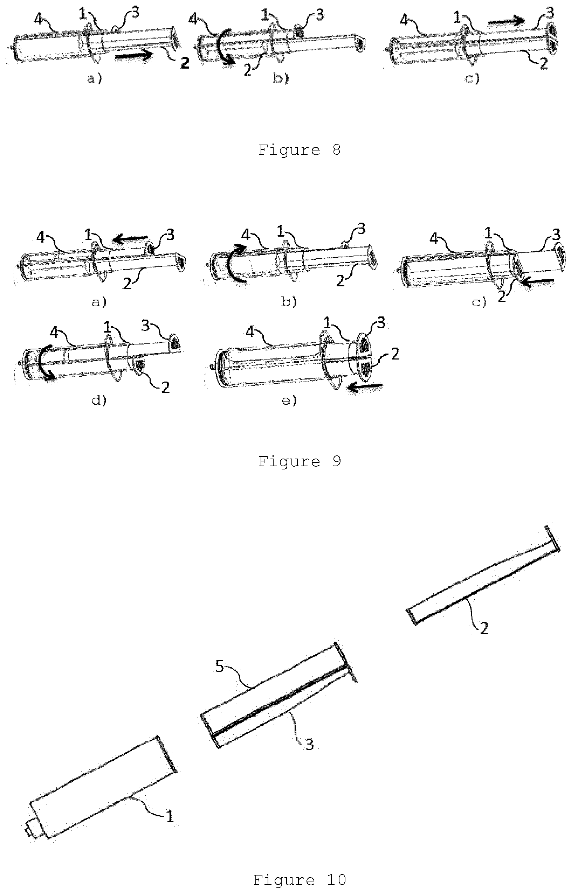

Figure 8 - representation of the loading phase of the preferential embodiment shown in Figures 6 and 7. The syringe is placed in the initial position, which is equal to the final position (Fig. 8a), and the loading phase uses three movements: first movement to load the washing solution by moving the plunger (3) in the direction indicated in Fig. 8a), second movement to rotate the selector (4) to align with the orifice of the medicine chamber (Fig. 8b), and a third movement to load the medicine by moving the plunger (2) in the direction indicated in Fig 8c).
Figure 9 - representation of the administration phase in the preferential embodiment shown in Figures 6 and 7, this phase being achieved in five different movements: the pre-flushing with washing solution is performed by moving the plunger (3) Fig. 9a); the second movement is performed by rotating the selector (4) to the position of the medicine chamber Fig. 9b); in the third movement, the administration of the medicine is performed by moving the plunger (2) in the direction indicated in Fig. 9c); the fourth movement involves the rotation of the selector (4) again to the position of the washing solution chamber, Fig. 9d) and, at last, the final flushing by moving the plunger (3) in the direction indicated in Fig. 9e).

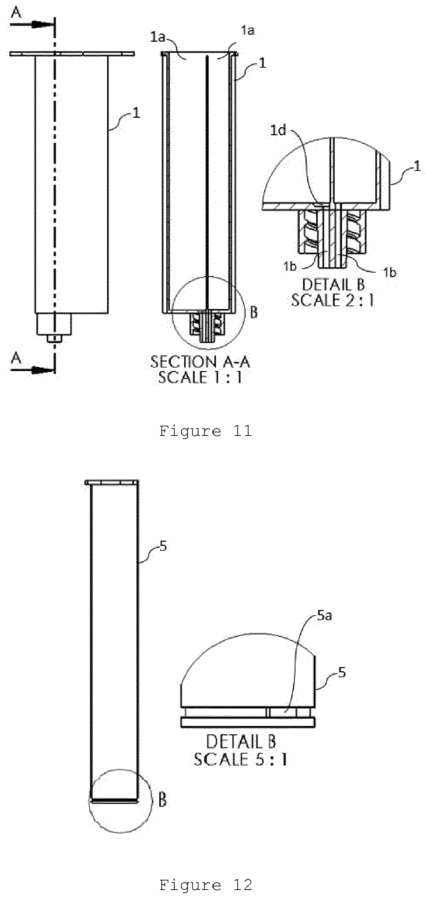

Figures 10, 11 and 12 - Representation of this invention in a third preferential embodiment consisting of four components, two independent chambers (1 and 5) and two plungers (3 and 2). One of the chambers (5) operates as a chamber/plunger. The chamber (1) comprises two independent compartments (1a), one for the medicine and one for the washing solution intended for pre-flushing. It further comprises an independent outlet (1b) for the washing solution used in the pre-flushing, i.e. there are two chambers: one chamber has an independent outlet for receiving the pre-flushing solution, while the other chamber has the washing solution for the final flushing and the medicine. One of the chambers (1a) has a small antechamber (1d) that connects with the other outlet (1b) through which the washing solution and the medicine are loaded/administered. In the chamber (5), referring to the chamber with dual purpose (chamber/plunger), an outlet (5a) can be observed that allows the fluid inside it to enter/leave the antechamber (1d) (Fig. 12) when in the loading/administration position.

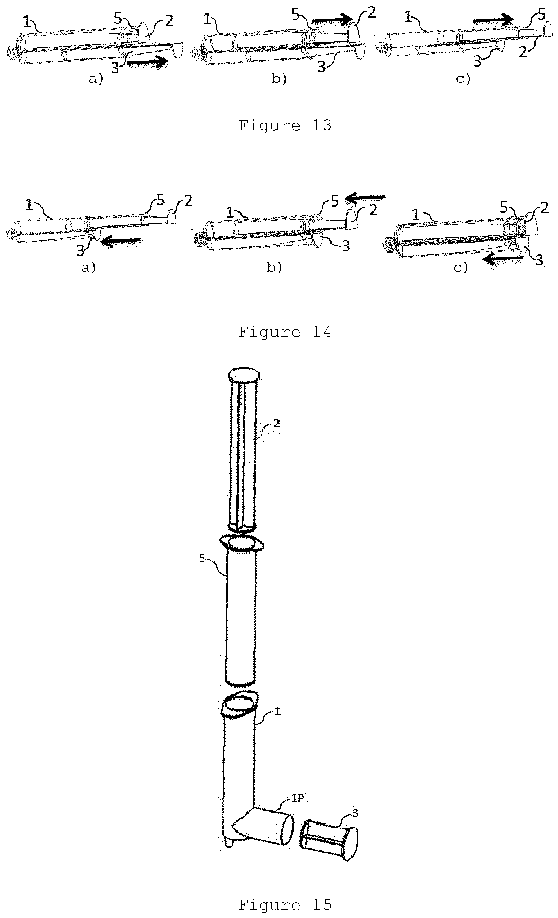

Figure 13 - representation of the loading phase in the preferential embodiment shown in Figures 10, 11 and 12. The syringe is placed in the initial position, and the loading phase is achieved using three movements: loading the pre-flushing washing solution by moving the plunger (3) in the direction indicated in Fig. 13a); loading the washing solution for the final flushing by moving the plunger (2) in the direction indicated in Fig. 13b); and finally, loading the medicine into the chamber's (1) compartment (1a) by moving the chamber/plunger (5) in the direction indicated in Fig. 13c).
Figure 14 - representation of the administration phase of the preferential embodiment shown in Figures 10, 11 and 12. This phase is carried out in two steps. The first step relates to pre-flushing, by moving the plunger (3) in the direction indicated in Fig. 14a), followed by the movement of the plunger (2) in the direction indicated in Fig. 14b), which, in its turn, by means of counter pressure, causes the chamber (5) to move, thus administering the medicine. The second step, following the first movement of the plunger (3), provides administration of the washing solution for the final flushing, Fig. 14c).

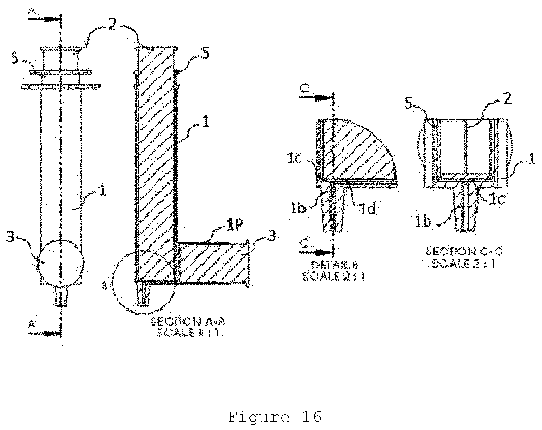

Figures 15, 16 and 17 - Representation of this invention in a fourth preferential embodiment consisting of five components, three independent chambers (1,5 and 1P) and two plungers (3 and 2). One of the chambers (5) has a dual purpose, operating as chamber/plunger. It has a third, perpendicular, chamber (1P) allowing the washing solution to be administered for the final flushing. The perpendicular chamber (1P) has a perpendicular outlet (1b), the said outlet being connected to a small antechamber (1d) which in turn contains an outlet (1b) through which the liquids are entered/expelled.

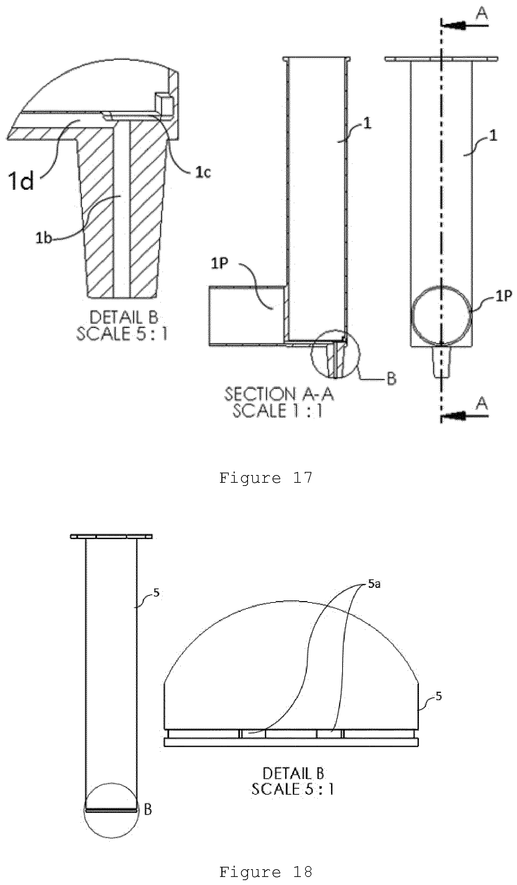

Figure 18 - The component (5), referring to the chamber with dual purpose (chamber/plunger), comprises two outlets (5a) that allow the liquid inside it to enter/leave to/from the antechamber (1d) (Fig. 17) when in the loading/administration position.

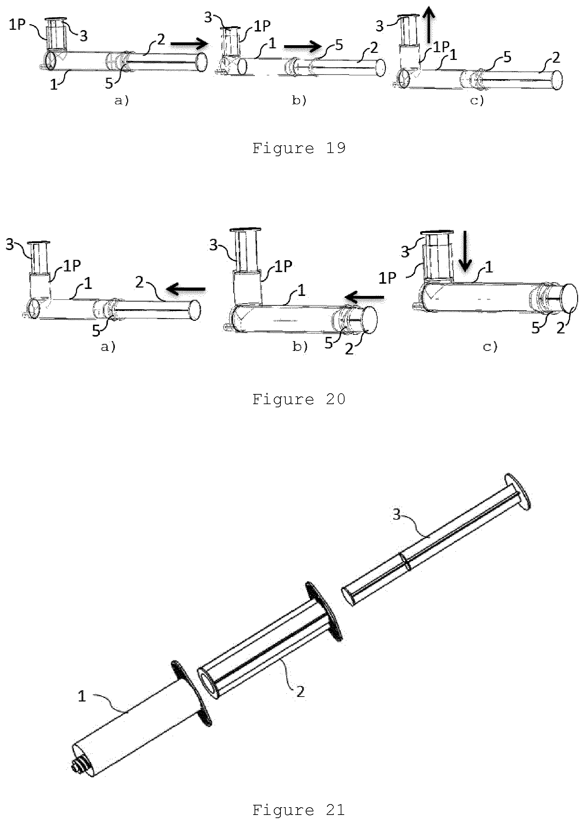

Figure 19 - representation of the loading phase in the preferential embodiment shown in Figures 15, 16, 17 and 18. The loading phase is achieved using three movements: loading the medicine by moving the plunger (2) in the direction indicated in Fig. 19a), followed by loading of the washing solution for pre-flushing into the chamber (1) through the chamber/plunger (5), moving it in the direction indicated in Fig. 19b), and finally loading the washing solution for final flushing into the chamber (1P) by moving the plunger (3) in the direction indicated in Fig. 19c).
Figure 20 - representation of the administration phase of the preferential embodiment shown in Figures 15 to 18. This phase is carried out in two distinct steps: the first step of the first movement is performed by moving the plunger (2) in the direction indicated in Fig. 20a) which, in its turn, by means of counterpressure, causes the component (5) to move, applying the washing solution for pre-flushing followed by the administration of the medicine (Fig. 20b), after the first movement of the plunger (2). The second step relates to the final flushing and is performed by using the movement of the plunger (3) in the direction indicated in Fig. 20c).

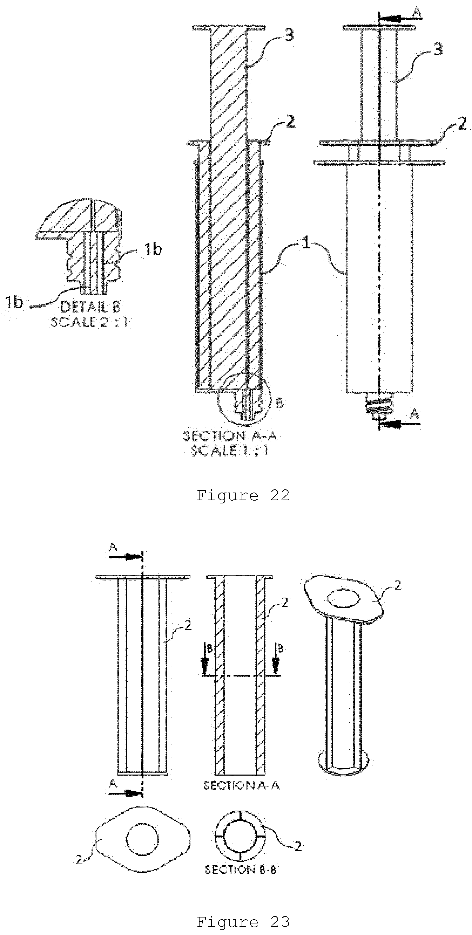

Figures 21, 22 and 23 - Representation of the present invention in a fifth and last preferential embodiment in which it consists of three independent components, the double chamber syringe (1) and two independent plungers (2 and 3). It comprises two independent outlets (1b), preventing the fluids to be mixed and allowing the administration of the washing solution (pre-flushing), administration of the medicine and again the administration of the washing solution (final flushing). The medicine plunger (2) consists of a central hole which allows the washing solution plunger (3) to pass through and ensures loading/administration without interfering with it.

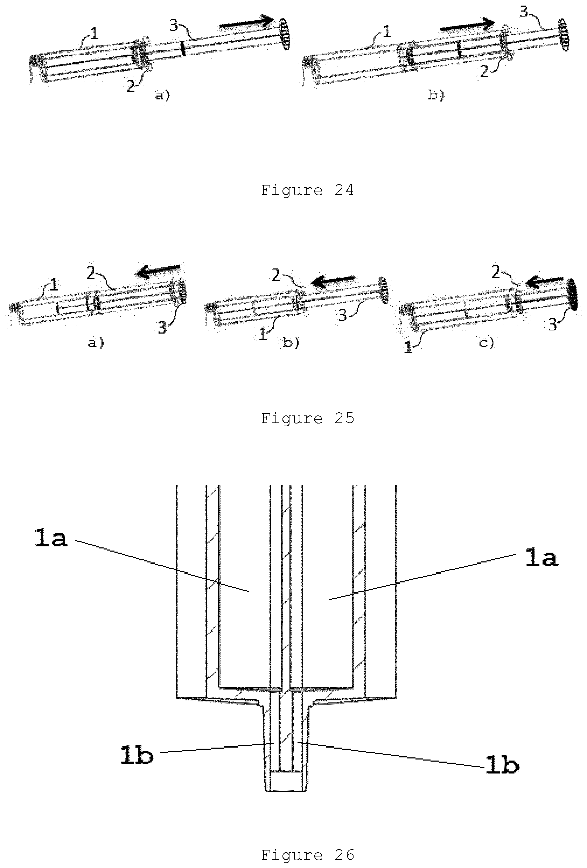

Figure 24 - representation of the loading phase in the preferential embodiment shown in Figures 21, 22 and 23, with the loading phase being achieved using two movements, loading the washing solution by moving the plunger (3) in the direction shown in Fig. 24a) and loading the medicine by moving the plunger (2) in the direction shown in Fig. 24b).
Figure 25 - representation of the administration phase in the preferential embodiment shown in Figures 21, 22 and 23. This phase is carried out in three different movements: pre-flushing with washing solution is performed by moving the plunger (3) Fig. 25a), the second movement is aimed at administering the medicine by moving the plunger (2) until the end of the total capacity of the respective chamber Fig. 25b); Finally, the administration of the washing solution for the final flushing is performed using the movement of the plunger (3) Fig. 25c).

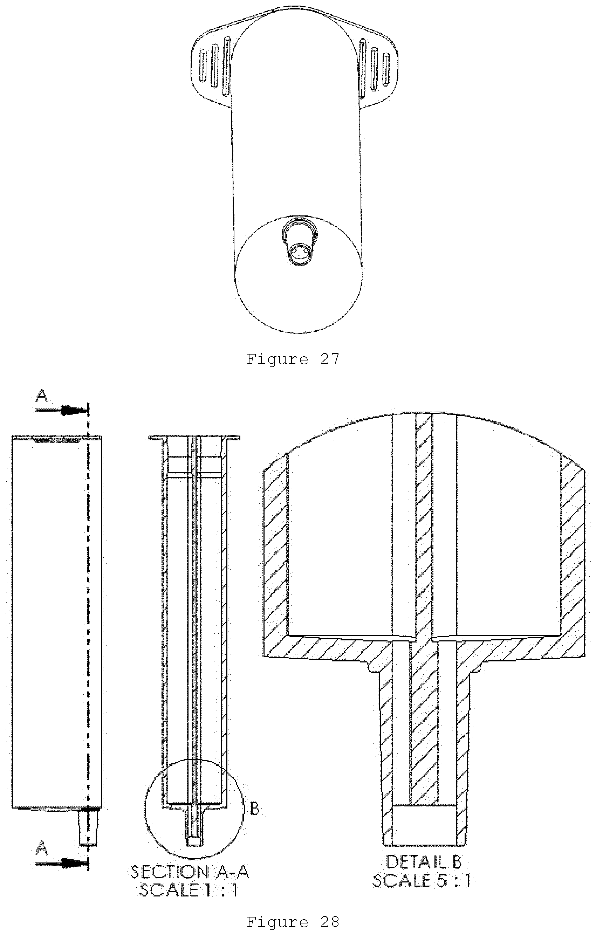

Figures 26, 27 and 28 - detailed representation of the end of the double chamber (1) showing the independent outlets (1b) and the independent compartments (1a).

### DETAILED DESCRIPTION OF THE INVENTION

This invention relates to a syringe with at least one double chamber (1) with independent outlets/inlets (1b); a medicine chamber independent plunger (2); a washing solution chamber independent plunger (3); and it may also have a selector (4); and/or an independent chamber (5), all of which are configured in such a way that the administration of the medicine is interspersed with two washes (pre-flushing and final flushing).

In order to better describe the invention, reference will be made to the enclosed Figures. However, they should not limit the scope of protection of this invention, but rather support its description.

This invention, in its basic embodiment and its first preferential embodiment (Figures 1 to 5), presents two independent chambers (1a) in the syringe's body (1), two independent plungers (2) (3), and two independent outlets (1b) preventing the mixing of fluids. In this preferred embodiment, two movements are performed for loading the syringe, one for loading the washing solution and the other for loading the medicine. The administration is carried out in three movements: firstly, the washing solution is administered to evaluate the permeability (pre-flushing), followed by the administration of the medicine and, then, a final washing of the catheter, needle or device that is connected to the syringe barrel (final flushing).

In a second preferential embodiment (Figures 6 to 9), the present invention comprises two chambers (1a), as in the previous embodiment, two plungers (2)(3) and a selector (4) that allows the rotation of the chamber according to the fluid to be administered. Each fluid exits through the same orifice in the selector (4a), however each of the chambers (1a) has an independent outlet. In this preferred embodiment three movements are performed for loading: one for loading the medication, followed by a rotation of the syringe using the selector (4) for alignment with the hole in the washing solution chamber and, finally, loading of the washing solution. The administration is carried out in five movements: evaluation of permeability (pre-flushing), rotation of the selector (4) to the medicine position, administration of the medicine, opposite rotation to the washing solution position and final flushing.

In a third preferential embodiment of the invention (Figures 10 to 14), the syringe comprises two independent chambers (1 and 5) and two plungers (3 and 2). The chamber (5) acts simultaneously as a plunger, and is located inside one of the compartments (1a). The chamber (1) comprises two independent compartments (1a), one for the medicine and one for the pre-flushing washing solution, and two independent outlets (1b), one for the serum used in the pre-flushing and the other through which the medicine and the washing solution for the final flushing are loaded/administered. One of the chambers (1a) has a small antechamber (1d) that connects to one of the outlets (1b). The chamber (5) has an outlet (5a) that allows the liquid inside it to enter/leave the antechamber (1d) (Fig. 12) when in the loading/administration position. In this preferred embodiment, three movements are also performed: loading the pre-flushing washing solution by moving the plunger (3), followed by loading of the final flushing solution into the chamber (5) via the plunger (2); finally loading the medicine into the chamber (1) compartment (1a) by moving the chamber (5). The administration is carried out in two distinct steps: the first step relates to the pre-flushing, by moving the plunger (3), the second movement being performed by moving the plunger (2) which in its turn, by means of counterpressure, causes the chamber (5) to move and administering the medicine, followed by the second step which, after the first movement of the plunger (3), administers the washing solution for the final flushing.

In a fourth preferential embodiment of the invention (Figures 15 to 20), the syringe comprises three independent chambers (1, 5 and 1P) and two plungers (3 and 2). The chamber (5) operates a chamber/plunger and has two outlets (5a) that allow the liquid inside to enter/leave the antechamber (1d). The said chamber (5) is interior and concentric with the chamber (1). It further comprises a third chamber (1P), which is perpendicular, allowing the washing solution to be administered for the final flushing. The perpendicular chamber (1P) has a perpendicular outlet (1b), connected to a small antechamber (1d) which in turn contains an outlet (1b) through which the liquids are entered/expelled. In this preferred embodiment, loading is carried out using three movements: loading the medicine by moving the plunger (2), followed by the washing solution being loaded into the pre-flushing chamber (1) through the chamber (5), and loading the washing solution for the final flushing into the chamber (1P) by moving the plunger (3). The administration includes two distinct movements: the first step of the first movement is carried out by moving the plunger (2) which, by means of counterpressure, moves the component (5) applying the washing solution for the pre-flushing, followed by the second step which, after the first movement of the plunger (2), administers the medicine. The second movement is performed by moving the plunger (3) for administration of the washing solution for the final flushing.

The fifth and last preferential embodiment of the invention (Figures 21 to 25) relates to a syringe comprising three independent components, the syringe's body (1) with two independent outlets (1a) preventing the mixing of fluids, a plunger (2) consisting of a central hole allowing the washing solution plunger (3) to pass through, which ensures the loading/administration without interfering with it. In this preferred embodiment, loading is achieved with two movements: loading the washing solution by moving the plunger (3) and loading the medicine by moving the plunger (2). The administration takes place with three different movements: the administration of the washing solution (pre-flushing) is performed by moving the plunger (3); then, a second movement is performed for the administration of the medicine by moving the plunger (2); and finally, moving the plunger (3), the washing solution is administered for the final flushing.

It shall be noted that all the plungers of this invention may have o-rings at their ends for a better retention of the substances contained in the respective chambers.

## Claims

1. A syringe **characterized in that** it comprises:
a) at least one double chamber (1) with two independent outlets;
b) at least two independent plungers (2)(3);
c) the chamber (1) and the plungers (2)(3) are configured to the administration of the medicine being interspersed with two washes.

2. A syringe according to claim 1, wherein the double chamber (1) comprises two independent compartments (1a), one for the medicine and one for the washing solution, also having two independent outlets (1b) preventing the mixing of fluids.

3. A syringe according to the previous claim, wherein further comprises a selector (4) coating the chamber (1) and over which the chamber (1) slides, said selector (4) having an outlet (4a) promoting the rotation of the chamber (1) depending on the fluid to be administered.

4. A syringe according to claims 1 and 2, wherein further comprises a chamber (5) located inside one of the compartments (1a) of the chamber (1), the other compartment (1a) having an antechamber (1d) connected to one of the outlets (1b), and the chamber (5) having an outlet (5a) connected to the antechamber (1d).

5. A syringe according to claim 1, **characterized in that** the double chamber (1) comprises two concentric independent compartments: the chamber (1) and a chamber (5) which is inside the chamber (1), said chamber (5) having two outlets (5a) connected to an antechamber (1d); and **in that** the syringe also comprises a chamber (1P) perpendicular to the chamber (1), which has an outlet (1b), the said outlet (1b) being connected to an antechamber (1d).

6. A syringe according to claim 1, **characterized in that** the double chamber (1) comprises two concentric independent compartments: the chamber (1) and the medicine plunger (2), said plunger (2) being hollow and the serum plunger (3) sliding inside the plunger (2).
